# EUROPEAN PATENT APPLICATION

(11) **EP 2 241 259 A1**
(43) Date of publication of application: **20.10.2010**
(21) Application number: 10157888.8
(22) Date of filing: 26.03.2010
(51) Int. Cl.: A61B 8/08

(54) **Ultrasonic diagnostic apparatus**

(30) Priority: 14.04.2009 KR 20090032265
(71) Applicant: Medison Co., Ltd., Gangwon-do 250-875 (KR)
(72) Inventor: Kim, Seong Rae, Gyeonggi-do (KR); Hyun, Dong Gyu, Gyeonggi-do (KR); Kim, Chul An, Gyeonggi-do (KR); Park, Hee Jung, Seoul (KR)
(74) Representative: Schmid, Wolfgang

(57) **Abstract**

An ultrasonic diagnostic apparatus is disclosed. The ultrasonic diagnostic apparatus (100) includes (110; 210; 310) exposing one side of a diagnosis target (10) while supporting the diagnosis target (10) so as to maintain a shape of the diagnosis target (10), an oblique part (120; 220; 320) obliquely formed on the housing (110; 210; 310) to support the diagnosis target (10), and a probe unit (130; 330) disposed inside the housing (110; 210; 310) to examine the diagnosis target (10). The ultrasonic diagnostic apparatus (100) ensures efficient diagnosis of the overall diagnosis target without excessive compression of the diagnosis target (10) causing examinee discomfort, and can support the diagnosis target (10) so as to maintain the shape of the diagnosis target, thereby ensuring the provision of a constant quality of ultrasonic images upon repetitious diagnosis of the diagnosis target (10) while improving reproducibility of diagnosis results.

## Description

### BACKGROUND OF THE INVENTION

### 1. Field of the Invention

The present invention relates to an ultrasonic diagnostic apparatus and, more particularly, to an ultrasonic diagnostic apparatus that performs diagnosis for a target using ultrasound waves.

### 2. Description of the Related Art

Breast cancer is not only the most common type of cancer in the West, but also has a very high incidence rate together with cervical cancer and stomach cancer among Korean women. Primary diagnosis of breast cancer is generally performed using an X-ray imaging system. The X-ray imaging system is very convenient for diagnosis and thus has been widely used in the art. However, the X-ray imaging system has a very low diagnosis rate for dense tissue of the breast.

The X-ray imaging system has significantly low effectiveness and a very high misdiagnosis rate in diagnosis of breast cancer, particularly for Korean women who generally have much denser breast tissue than Western women.

Recently, an ultrasonic diagnostic apparatus has been employed instead of the X-ray imaging system. The ultrasonic diagnostic apparatus does not provide a danger of radiation exposure and is capable of processing images of a diagnosis target such as three-dimensional images and detecting small cancers of about 2∼3 mm.

The ultrasonic diagnostic apparatuses for diagnosis of breast cancers are generally classified into a supine type ultrasonic diagnostic apparatus configured to diagnose an examinee lying face up, a prone type ultrasonic diagnostic apparatus configured to diagnose an examinee lying face down, and an upright type ultrasonic diagnostic apparatus configured to diagnose an examinee standing or sitting therein.

Since the supine type and prone type ultrasonic diagnostic apparatuses require an examinee to lie face up or face down during diagnosis, they occupy a large installation space and entail a very complicated diagnosis procedure, thereby causing low diagnosis efficiency. Furthermore, in these types of ultrasonic diagnostic apparatuses, the examinee is diagnosed in an inconvenient posture and is thus likely to suffer fatigue during diagnosis.

Since the upright type ultrasonic diagnostic apparatus allows an examinee to stand or sit during diagnosis, it occupies a smaller installation space and provides a more convenient diagnosis procedure than the other types of ultrasonic diagnostic apparatuses.

The upright type ultrasonic diagnostic apparatus is provided with a compression rack that includes probes and performs diagnosis of an examinee in a standing or sitting posture while compressing a diagnosis target of the examinee up and down or right and left.

As such, since the upright type ultrasonic diagnostic apparatus performs the diagnosis while compressing the diagnosis target, not only does the examinee suffer pain, but the shape of the diagnosis target is also changed depending on a degree of compressing the target to provide different images of the diagnosis target, thereby deteriorating reproducibility of diagnosis results.

Further, in such an upright type ultrasonic diagnostic apparatus, the shape of the diagnosis target is changed upon compression, so that the overall diagnosis target does not come into close contact with the compression rack. As a result, diagnosis is carried out only for part of the diagnosis target closely contacting the compression rack and the other parts of the diagnosis target are not subjected to diagnosis. Therefore, there is a need to solve such a problem.

### SUMMARY OF THE INVENTION

The present invention is conceived to solve the problem of the related art, and an aspect of the invention is to provide an improved ultrasonic diagnostic apparatus that can improve reproducibility of diagnosis results and ensure efficient diagnosis for a diagnosis target of an examinee while reducing examinee discomfort.

In accordance with one aspect of the invention, an ultrasonic diagnostic apparatus includes: a housing exposing one side of a diagnosis target while supporting the diagnosis target so as to maintain a shape of the diagnosis target; an oblique part obliquely formed on the housing to support the diagnosis target; and a probe unit disposed inside the housing to examine the diagnosis target.

The oblique part may have an upward slope corresponding to the shape of the diagnosis target from a lower side of the oblique part to an upper side thereof.

The oblique part may have a linear cross-section in one direction and may have a curved cross-section in a direction in which the probe unit moves.

The oblique part may include a flexible portion that is deformed when the diagnosis target comes into contact with the flexible portion.

The probe unit may move along a path including a curved path in a longitudinal direction of the oblique part to examine the diagnosis target.

The probe unit may be obliquely disposed corresponding to the oblique part.

The probe unit may include a guide member formed to include a curved shape and an ultrasound probe coupled to the guide member to be moved thereon.

The diagnosis target may be a breast of an examinee, the oblique part may have a length supporting both breasts of the examinee, and the probe unit may move along a path including a curved path to examine both breasts of the examinee through a single examination operation.

The probe unit may include a plurality of ultrasound probes.

The ultrasonic diagnostic apparatus may further include a lift part raising or lowering the housing.

The ultrasonic diagnostic apparatus may further include an oblique movement part obliquely moving the housing to approach or move away from the diagnosis target.

The ultrasonic diagnostic apparatus may be an upright type ultrasonic diagnostic apparatus.

### BRIEF DESCRIPTION OF THE DRAWINGS

The above and other aspects, features and advantages of the invention will become apparent from the following description of embodiments given in conjunction with the accompanying drawings, in which:
Fig. 1 is a perspective view of an ultrasonic diagnostic apparatus in accordance with a first embodiment of the present invention;
Fig. 2 is a view of one exemplary use of the ultrasonic diagnostic apparatus in accordance with the first embodiment of the present invention;
Fig. 3 is a cross-sectional view taken along line A-A of Fig. 2;
Fig. 4 is a cross-sectional view taken along line B-B of Fig. 2;
Fig. 5 is a side view of an oblique movement part of the ultrasonic diagnostic apparatus in accordance with the first embodiment of the present invention;
Figs. 6 and 7 are side views of a lift part of the ultrasonic diagnostic apparatus in accordance with the first embodiment of the present invention;
Fig. 8 is a view of another exemplary use of the ultrasonic diagnostic apparatus in accordance with the first embodiment of the present invention;
Fig. 9 is a perspective view of a housing of an ultrasonic diagnostic apparatus in accordance with a second embodiment of the present invention;
Figs. 10 and 11 are cross-sectional views of the housing of the ultrasonic diagnostic apparatus in accordance with the second embodiment of the present invention;
Fig. 12 is a perspective view of a housing of an ultrasonic diagnostic apparatus in accordance with a third embodiment of the present invention; and
Fig. 13 is a cross-sectional view of the housing in accordance with the third embodiment of the present invention.

### DETAILED DESCRIPTION OF THE EMBODIMENT

Embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defmed by taking functions of the invention into account and can be changed according to the custom or intention of users or operators. Therefore, definition of the terms should be made according to overall disclosures set forth herein.

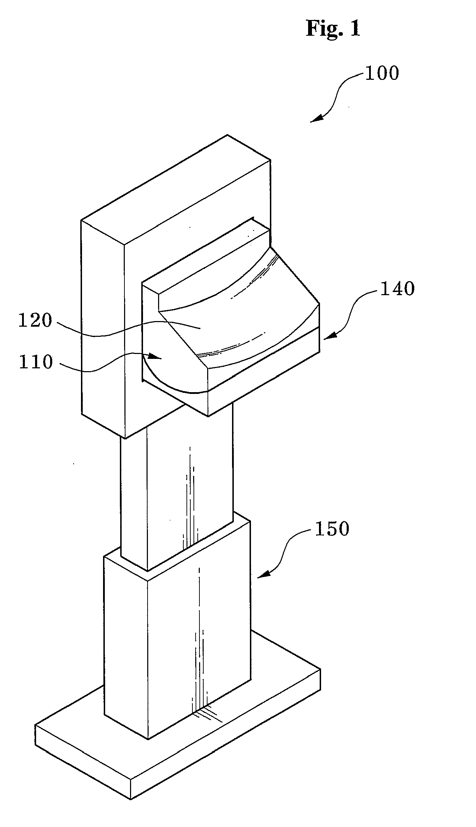

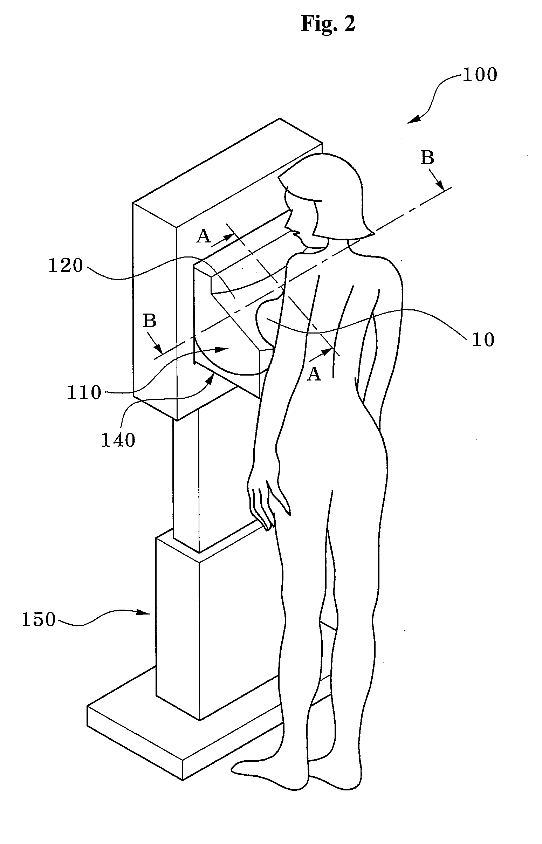

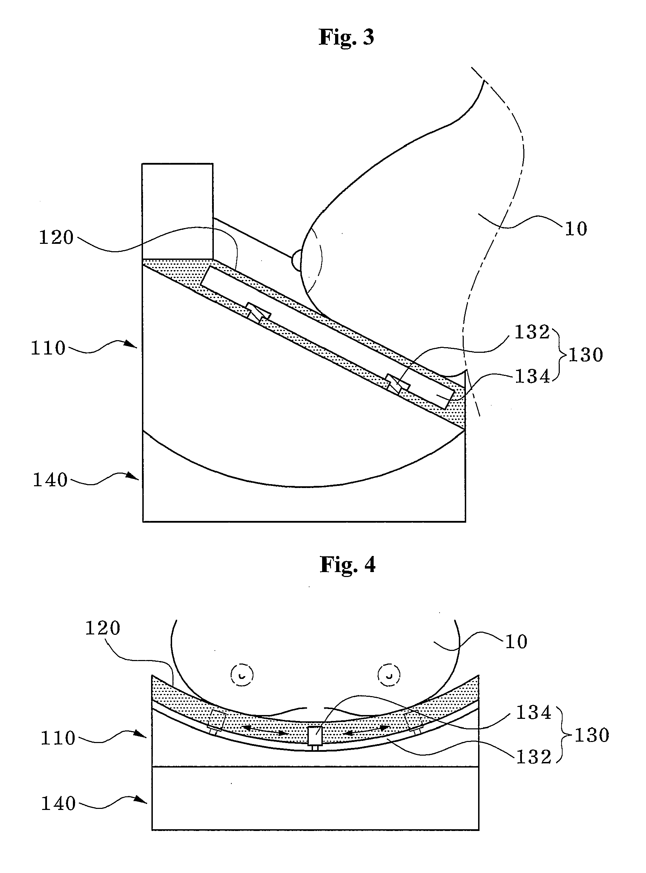

Fig. 1 is a perspective view of an ultrasonic diagnostic apparatus in accordance with a first embodiment of the invention, Fig. 2 is a view of one exemplary use of the ultrasonic diagnostic apparatus in accordance with the first embodiment, Fig. 3 is a cross-sectional view taken along line A-A of Fig. 2, and Fig. 4 is a cross-sectional view taken along line B-B of Fig. 2.

Referring to Figs. 1 to 4, an ultrasonic diagnostic apparatus 100 according to this embodiment may be an upright type breast ultrasonic diagnostic apparatus that can carry out ultrasound diagnosis for a diagnosis target of an examinee in a standing or sitting posture. The ultrasonic diagnostic apparatus 100 includes a housing 110, an oblique part 120, and a probe unit 130.

The housing 110 supports a diagnosis target 10. The housing 110 may be filled with a fluid that facilitates transmission of ultrasound waves. The fluid in the housing 110 may be oil or the like. In the housing 110, an ultrasound probe 134 of the probe unit 130 is immersed in the fluid.

In this embodiment, the housing 110 supports the diagnostic target 10 and is configured to expose one side, for example, an upper side, of the diagnostic target 10. As such, the housing 110 exposes the upper side of the diagnostic target 10 to secure a visual field of an operator so that the operator can check the position and state of the diagnosis target with the naked eye from above the upper side of the diagnosis target 10.

Further, the housing 110 supports the diagnosis target 10 so as to "maintain" the shape of the diagnosis target 10 while exposing the one side of the diagnosis target 10. Herein, the term "maintain" means that the housing 110 supports only one side of the diagnosis target 10 and does not compress the diagnosis target 10 to such a degree that an examinee experiences discomfort.

According to this embodiment, the diagnosis target 10 is illustrated as being the breast of a person. The diagnosis target 10 such as the breast is likely to be deformed upon compression.

In this embodiment, the housing 110 supports only the other side of the diagnosis target 10, for example, a lower side of the diagnosis target 10, thereby supporting the diagnosis target 10 without excessive compression of the diagnosis target 10. As a result, the diagnosis target 10 can be supported by the housing 110 without undergoing substantial shape change.

The oblique part 120 is obliquely formed on the housing 110 to support the diagnosis target 10. The oblique part 120 will directly contact the diagnosis target 10 while supporting the diagnosis target 10. The oblique part 120 has an upward slope corresponding to the shape of the diagnosis target 10 from a lower side of the oblique part 120 to an upper side thereof.

The oblique part 120 may have a length capable of supporting the diagnosis target 10, for example, both breasts of an examinee. Further, the oblique part 120 has a "linear shape" cross-section orthogonal to a longitudinal direction of the oblique part 120 (see Fig. 3) and a "curved shape" cross-section in a direction in which the probe unit 130 described below moves, that is, in the longitudinal direction of the oblique part 120 (see Fig. 4).

Herein, the "linear shape" cross-section of the oblique part 120 includes not only a completely linear shape but also a substantially linear shape, the overall shape of which approaches the linear shape. Further, the "curved shape" cross-section in the longitudinal direction of the oblique part 120 may be a curved shape similar to the shape of the diagnosis target 10, which will come into contact with the oblique part 120.

The probe unit 130 is disposed inside the housing 110. The probe unit 130 is movably disposed inside the housing 110 to examine the diagnosis target 10.

Further, the probe unit 130 may be obliquely disposed corresponding to the oblique part 120 and move along a path including a curved path defined in the longitudinal direction of the oblique part 120 so as to examine both breasts of an examinee supported on the oblique part 120 through a single diagnosis operation. The probe unit 130 includes a guide member 132 and an ultrasound probe 134.

The guide member 132 is formed to include a curved shape. The guide member 132 may be obliquely disposed corresponding to the oblique part 120 and may be formed to include a curved shape corresponding to the longitudinal shape of the oblique part 120.

The ultrasound probe 134 is coupled to the guide member 132 and moved thereon. The ultrasound probe 134 is provided with a transducer (not shown), which transmits an ultrasound signal to the diagnosis target 10 and receives the ultrasound echo-signal reflected from the diagnosis target 10, and reciprocates on the rear side of the oblique part 120 along a path defined by the guide member 132.

The ultrasound probe 134 examines the diagnosis target 10 by sending an ultrasound signal to the diagnosis target 10 and receiving the ultrasound echo-signal reflected from the diagnosis target 10, in which the ultrasound signal sent from the ultrasound probe 134 or reflected from the diagnosis target 10 is transmitted to the diagnosis target or the ultrasound probe 134 via the fluid having the ultrasound probe 134 immersed therein.

In this embodiment, the probe unit 130 may include a single or plurality of ultrasound probes 134.

When the probe 130 includes a single ultrasound probe 134, the ultrasound probe 134 of the probe 130 may have a width capable of examining the diagnosis target 10 over the entire width of the oblique part 120 to examine the overall diagnosis target 10 while moving in the longitudinal direction of the oblique part 120.

When the probe unit 130 includes two ultrasound probes 134, the ultrasound probes 134 of the probe unit 130 may be arranged parallel to each other or so as to cross each other in the width direction of the oblique part 120.

When the ultrasound probes 134 are arranged parallel to each other in the width direction of the oblique part 120, the combination of the ultrasound probes 134 can examine the overall diagnosis target 10 as in the case where the probe unit 130 includes a single ultrasound probe 134.

When the ultrasound probes 134 are arranged so as to cross each other in the width direction of the oblique part 120, each of the ultrasound probes 134 can examine the corresponding area of the diagnosis target while moving at different locations.

The ultrasonic diagnostic apparatus 100 according to this embodiment may further include an oblique movement part 140.

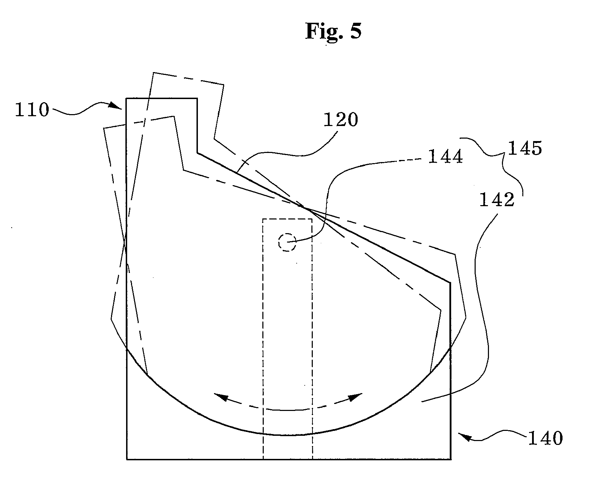

Fig. 5 is a side view of an oblique movement part of the ultrasonic diagnostic apparatus in accordance with the first embodiment.

Referring to Fig. 5, the oblique movement part 140 obliquely moves the housing 110 such that the housing 110 approaches or moves away from the diagnosis target 10. The oblique movement part 140 includes a hinge 145 and a slope controller (not shown).

The hinge 145 pivotably couples the housing 110 to the oblique movement part 140, and includes a frame 142 for supporting the housing 110 and a hinge shaft 144 for pivotably coupling the housing 110 to the frame 142.

The slope controller restricts pivoting of the housing 110 around the hinge 145. The slope controller includes a drive motor which generates a drive force for pivoting the housing 110, and a power transmission which transmits the drive force from the drive motor to the housing 110 so that the housing 110 pivots around the hinge 145. The drive motor of the slope controller may be a stepper motor. Such configuration of the slope controller is apparent to a person having ordinary knowledge in the art and a detailed description thereof will be omitted herein.

The housing 110 is obliquely moved by the oblique movement part 140 to approach or move away from the diagnosis target 10 (see Fig. 3), so that the position of the housing 110 can be adjusted depending on the size or shape of the diagnosis target 10.

With the housing 110 obliquely moved away from the diagnosis target 10, the oblique part 120 can stably support the diagnosis target 10 having a large size without shape change of the diagnosis target 10. With the housing obliquely moved to approach the diagnosis target 10, the oblique part 120 can stably support the diagnosis target 10 having a small size without the shape change of the diagnosis target 10.

In this embodiment, the ultrasonic diagnostic apparatus 100 may further include a lift part 150.

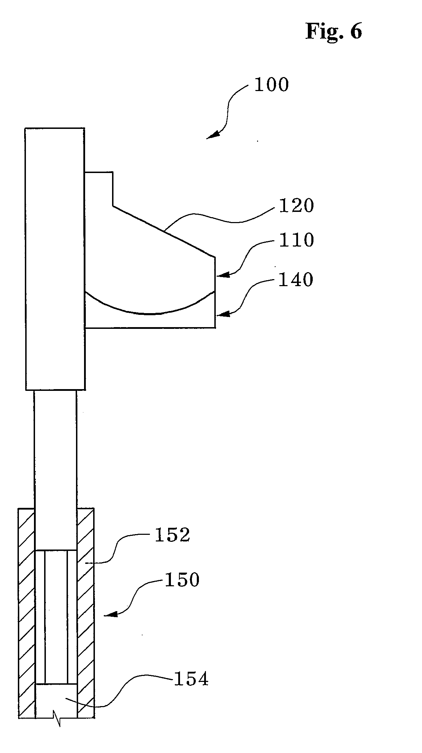

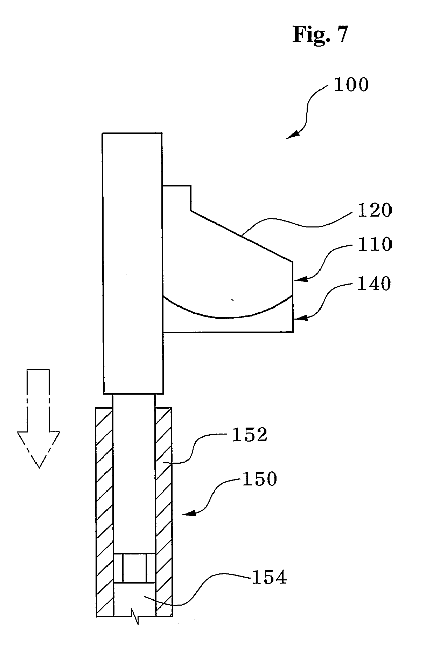

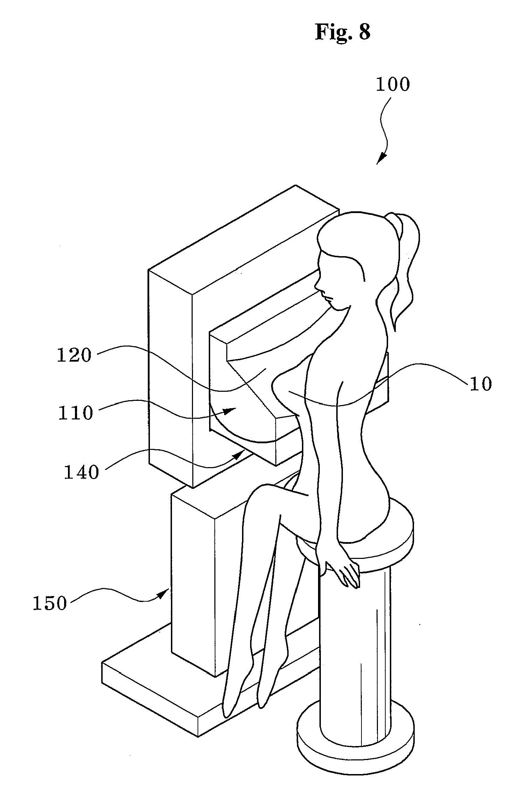

Figs. 6 and 7 are side views of a lift part of the ultrasonic diagnostic apparatus in accordance with the first embodiment, and Fig. 8 is a view of another exemplary use of the ultrasonic diagnostic apparatus in accordance with the first embodiment.

First, referring to Fig. 6, the lift part 150 raises or lowers the housing 110 and includes a support 152 and a drive unit 154.

The support 152 is disposed below the housing 110 and supports the housing 110 when the housing 110 is raised or lowered by the lift part. The support 152 may be coupled to the center or both sides of the housing 110.

The drive unit 154 is disposed in the support 152 and generates a drive force for raising or lowering the housing 110. The drive unit 154 may include an actuator that generates the drive force in the up-down direction to raise or lower the housing 110. Details of the drive unit are apparent to a person having ordinary knowledge in the art and a description thereof will be omitted herein.

As shown in Fig. 7, the lift part 150 raises or lowers the housing 110 to make an upper surface of the oblique part 120 coincident with the height of the diagnosis target 10 so that the diagnosis target 10 can be stably supported by the oblique part 120 not only when an examinee is in a standing posture but also in a sitting posture, as shown in Fig. 8.

Next, operation and effect of the ultrasonic diagnostic apparatus according to this embodiment will be described with reference to Figs. 1 to 8.

When performing ultrasound diagnosis using the ultrasonic diagnostic apparatus 100 according to this embodiment, a diagnosis target 10 of an examinee is supported on the oblique part 120, with the examinee in a standing posture or sitting posture. Here, if the diagnosis target 10 is the breasts of the examinee, it is desirable to manipulate the apparatus such that both breasts of the examinee are supported by the oblique part 120 and substantially most of the breasts come into close contact with the oblique part 120.

For this purpose, as shown in Figs. 6 and 7, the lift part 150 is activated to adjust the upper surface of the oblique part 120 to be positioned at a height coincident with the height of the breasts by raising or lowering the housing 110. At the same time, as shown in Fig. 5, the oblique movement part 140 is activated to adjust the position of the housing 110 to make the shape of the upper surface of the oblique part 120 close to the sizes and shapes of the breasts by obliquely moving the housing 110 to approach or move away from the breasts.

Not only can the housing 110 that is adjustable in height and position as described above stably support both breasts of an examinee, but also allows substantially most of the breasts to come into close contact with the oblique part 120, as shown in Figs. 3 and 4. Thus, the ultrasonic diagnostic apparatus 100 according to this embodiment as shown in Figs. 1 and 2 does not need to excessively compress the breasts in order to allow the breasts to come into close contact with the oblique part 120.

Further, the housing 110 that is adjustable in height and position as above allows the breasts to come into close contact with the oblique part 120 by adjusting the shape of the upper surface of the oblique part 120 to be close to the sizes and shapes of the breasts without compressing the breasts, so that the breasts can come into close contact with the oblique part 120 while maintaining the original shape thereof.

When the diagnosis target 10 is supported on the oblique part 120 as above, ultrasound diagnosis can be performed upon the diagnosis target 10 as shown in Fig. 4.

The ultrasound probe 134 in the housing 110 moves along the guide member 132 that may be formed to include a curved shape, for example, a curved shape corresponding to the longitudinal shape of the oblique part 120. The ultrasound probe 134 examines the diagnosis target 10 while moving along a path including the curved path corresponding to the longitudinal shape of the oblique part 120.

Here, although the diagnosis target 10, that is, both breasts, has a curved shape, the entirety of the diagnosis target 10 is brought into close contact with the oblique part 120 while maintaining the original shape thereof. Thus, the ultrasound probe 134 can obtain continuous images of internal tissue of the breast while reciprocating in the housing 110 and can examine both breasts through a single reciprocation, thereby enabling rapid diagnosis of the diagnosis target 10.

As described above, the ultrasonic diagnostic apparatus 100 according to this embodiment can allow most of the diagnosis target 10 to come into close contact with the oblique part 120 without excessive compression of the diagnosis target 10, thereby enabling efficient diagnosis of the overall diagnosis target 10 without causing discomfort to an examinee due to compression of the diagnosis target 10. Additionally, the ultrasonic diagnostic apparatus 100 allows the diagnosis target 10 to be supported while maintaining the shape thereof, thereby enabling the provision of a constant quality of ultrasound images obtained by repetitious diagnosis while improving reproducibility of diagnosis results.

Furthermore, since the ultrasonic diagnostic apparatus 100 can diagnose the overall diagnosis target 10 through a single diagnosis operation, it is possible to reduce inconvenience of an examinee through rapid diagnosis.

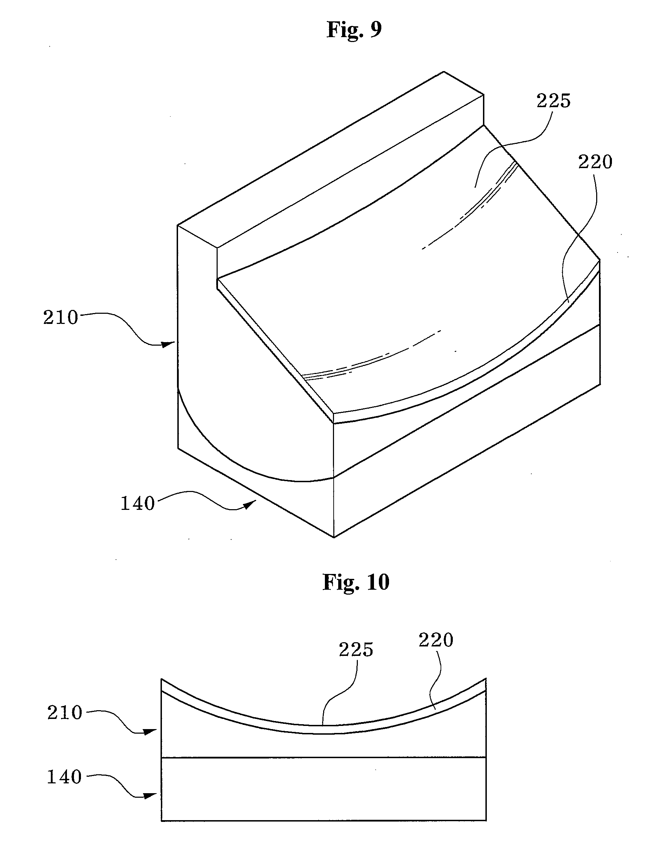

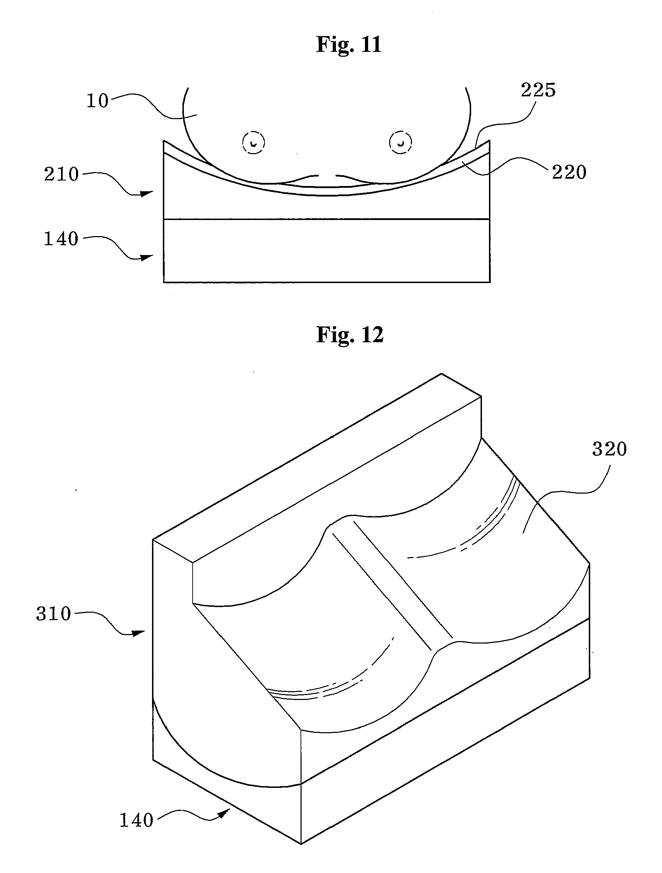

Fig. 9 is a perspective view of a housing of an ultrasonic diagnostic apparatus in accordance with a second embodiment of the invention, and Figs. 10 and 11 are cross-sectional views of the housing of the ultrasonic diagnostic apparatus in accordance with the second embodiment of the invention.

The same or similar components to those of the above embodiment will be denoted by the same reference numerals and detailed descriptions thereof will be omitted herein.

First, referring to Figs. 9 to 11, a housing 210 according to the second embodiment includes an oblique part 220.

In this embodiment, the oblique part 220 includes a flexible portion 225. The flexible portion 225 is disposed on the oblique part 220 and is deformed when a diagnosis target 10 comes into close contact therewith, as shown in Fig. 11. The flexible portion 225 may be formed of an elastic material that does not impede progress of ultrasound waves. For example, the flexible portion 225 may include a medium pocket that is filled with a material such as a gel, which will be applied to the diagnosis target upon ultrasound diagnosis.

When supporting the diagnosis target 10, the oblique part 220 including the flexible portion 225 allows the diagnosis target 10 to come into closer contact with the oblique part 220 while being maintained closer to the original shape thereof.

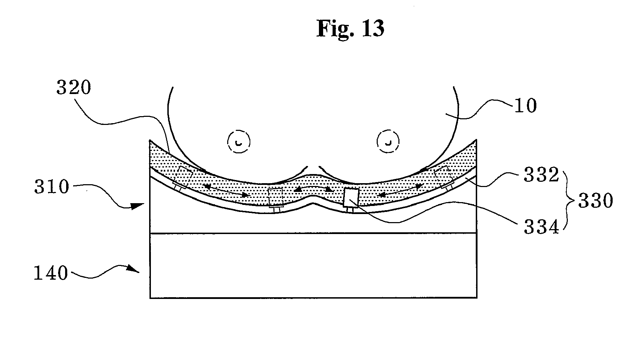

Fig. 12 is a perspective view of a housing of an ultrasonic diagnostic apparatus in accordance with a third embodiment of the invention, and Fig. 13 is a cross-sectional view of the housing in accordance with the third embodiment,

Referring to Figs. 12 and 13, a housing 310 of the ultrasonic diagnostic apparatus in accordance with the third embodiment includes an oblique part 320.

In this embodiment, the oblique part 320 has a substantially "UU" shape in the longitudinal direction thereof. As such, the oblique part 320 is formed in a shape similar to that of the diagnosis target 10, that is, both breasts of an examinee, to further reduce an area of the diagnosis target 10 that does not come into close contact with the oblique part 320, thereby allowing the diagnosis target 10 to come into closer contact with the oblique part 320 while being maintained closer to the original shape thereof.

In addition, a probe unit 330 is disposed in the housing 310 to move along a path including a "UU" shape path corresponding to a longitudinal shape of the oblique part 320.

In other words, a guide member 332 is formed to include the "UU" shape corresponding to the longitudinal shape of the oblique part 320, and an ultrasound probe 334 examines the diagnosis target 10 while moving along the path formed in the guide member 332 and including the "UU" shape, thereby further improving the quality of ultrasound images obtained by diagnosis.

As apparent from the above description, according to the embodiment, the ultrasonic diagnostic apparatus allows most of one side of a diagnosis target to come into close contact with the oblique part without excessive compression of the diagnosis target causing examinee discomfort, thereby preventing the examinee from suffering discomfort upon compression of the diagnosis target while ensuring efficient diagnosis of the overall diagnosis target.

In addition, according to the embodiment, the ultrasonic diagnostic apparatus can support the diagnosis target while maintaining the shape of the diagnosis target, thereby ensuring the provision of a constant quality of ultrasonic images upon repetitious diagnosis of the diagnosis target while improving reproducibility of diagnosis results.

Further, according to the embodiment, the ultrasonic diagnostic apparatus can diagnose the diagnosis target through a single diagnosis operation, thereby reducing inconvenience of an examinee through rapid diagnosis of the diagnosis target.

Further, according to the embodiment, the housing of the ultrasonic diagnostic apparatus exposes one side of the diagnosis target to secure a visual field of an operator, so that the operator can check the position and state of the diagnosis target with the naked eye from above, for example, an upper side of the diagnosis target.

Moreover, according to the embodiment, the housing can be raised and lowered to make the upper surface of the oblique part coincident with the height of the diagnosis target, so that the diagnosis target can be stably supported by the oblique part when an examiner is not only in a sitting posture but also in a standing postures.

Although some embodiments have been provided to illustrate the invention in conjunction with the drawings, it will be apparent to those skilled in the art that the embodiments are given by way of illustration only, and that various modifications and equivalent embodiments can be made without departing from the spirit and scope of the invention. The scope of the invention should be limited only by the accompanying claims.

## Claims

1. An ultrasonic diagnostic apparatus (100), **characterized by** comprising:
a housing (110; 210; 310) exposing one side of a diagnosis target (10) while supporting the diagnosis target (10) so as to maintain a shape of the diagnosis target (10);
an oblique part (120; 220; 320) obliquely formed on the housing (110; 210; 310) to support the diagnosis target (10); and
a probe unit (130; 330) disposed inside the housing (110; 210; 310) to examine the diagnosis target (10).

2. The apparatus (100) according to claim 1, **characterized in that** the oblique part (120; 220; 320) has an upward slope corresponding to the shape of the diagnosis target (10) from a lower side of the oblique part (120; 220; 320) to an upper side thereof.

3. The apparatus (100) according to claim 1, **characterized in that** the oblique part (120; 220; 320) has a linear cross-sectional in one direction and a curved cross-section in a direction in which the probe unit (130; 330) moves.

4. The apparatus (100) according to claim 1, **characterized in that** the oblique part (220) comprises a flexible portion (225) that is deformed when the diagnosis target (10) comes into contact with the flexible portion (225).

5. The apparatus (100) according to claim 1, **characterized in that** the probe unit (130; 330) moves along a path including a curved path in a longitudinal direction of the oblique part (120; 220; 320) to examine the diagnosis target (10):

6. The apparatus (100) according to claim 5, **characterized in that** the probe unit (130; 330) is obliquely disposed corresponding to the oblique part (120; 220; 320).

7. The apparatus (100) according to claim 5, **characterized in that** the probe unit (130; 330) comprises a guide member (132; 332) formed to include a curved shape, and an ultrasound probe (134; 334) coupled to the guide member (132; 332) to be moved thereon.

8. The apparatus (100) according to claim 1, **characterized in that** the diagnosis target (10) is a breast of an examinee, the oblique part (120; 220; 320) has a length supporting both breasts of the examinee, and the probe unit (130; 330) moves along a path including a curved path to examine both breasts of the examinee through a single examination operation.

9. The apparatus (100) according to claim 1, **characterized in that** the probe unit (130; 330) comprises a plurality of ultrasound probes (134; 334).

10. The apparatus (100) according to claim 1, **characterized by** further comprising: a lift part (150) raising or lowering the housing (110; 210; 310).

11. The apparatus (100) according to claim 1, **characterized by** further comprising:
an oblique movement part (140) obliquely moving the housing (110; 210; 310) to approach or move away from the diagnosis target (10).

12. The apparatus (100) according to any one of claims 1 to 11, **characterized in that** the ultrasonic diagnostic apparatus (100) is an upright type ultrasonic diagnostic apparatus.
